# EUROPÄISCHE PATENTSCHRIFT

(11) **EP 2 543 349 B1**
(45) Veröffentlichungstag und Bekanntmachung des Hinweises auf die Patenterteilung: **09.10.2013**
(21) Anmeldenummer: 12172825.7
(22) Anmeldetag: 20.06.2012
(51) Int. Cl.: A61F 13/00, A61F 13/02

(54) **Wundabdeckung und Vorrichtung zum Absaugen von Wundsekreten**
Wound covering and device for draining wound secretions
Recouvrement de plaie et dispositif d'aspiration de sécrétions

(30) Priorität: 07.07.2011 DE 102011051657
(43) Veröffentlichungstag der Anmeldung: 09.01.2013
(73) Patentinhaber: WESIL GbR, 37327 Birkungen (DE)
(72) Erfinder: Ihle, Peter, 37327 Birkungen (DE); Weise, Steffen, 99755 Ellrich (DE)
(74) Vertreter: Carlsohn, Alexander

(56) Entgegenhaltungen:
- WO-A1-2010/142819
- US-A1- 2004 077 984

## Beschreibung

Die Erfindung betrifft eine Wundabdeckung, die einen Schaumstoffkörper und ein schlauch- und/oder rohrförmiges Verbindungselement umfasst. Sie betrifft ferner eine Vorrichtung zum Absaugen von Sekreten aus Wunden. Schließlich wird ein Kit beschrieben, das eine solche Wundabdeckung umfasst.

Wundsekrete sind überwiegend wässerige Absonderungen, die aufgrund einer Verletzung aus einer Wunde austreten können. Die Zusammensetzung der Wundsekrete, die vor allem aus den Zellzwischenräumen gespeist werden, kann stark variieren. Es enthält vorwiegend Beimengungen von Serumproteinen und Spuren von Blutinhaltsstoffen. Bei einer Infektion enthalten Wundsekrete überdies erhöhte Mengen an Makrophagen, die die Mikroorganismen beseitigen sollen. Dies kann mit der Entstehung von Eiter verbunden sein. Wird das Wundsekret nicht aus einer Wunde entfernt, kann dies zur Bildung von Abszessen führen.

Zum Entfernen von Wundsekreten aus Wunden werden in der Praxis verschiedene Vorrichtungen verwendet, denen die Anwendung von Unterdruck, der mittels einer Saugpumpe erzeugt wird, gemeinsam ist.

Aus DE 692 24 847 T2 ist eine Vorrichtung zur Behandlung von Gewebeschäden bekannt, die einen flächigen Abschnitt aus offenporigem Schaumstoffkörper sowie einen Schlauch mit einem Abschnitt umfasst, der in den Schaumstoffkörper eingeklebt ist. Ferner umfasst die Vorrichtung eine Klebefolie, die über dem Schlauch und dem Schaumstoffabschnitt angeordnet ist und an der Haut haftet, die die Wunde umgibt. Auf diese Weise wird eine Dichtung geschaffen, die das Erzeugen eines Vakuums mittels einer Saugpumpe ermöglicht. Die Vorrichtung soll im sterilen Zustand verpackt werden, wobei die Klebefolie getrennt von der Kombination aus Schaumstoff und Schlauch abgepackt wird.

Aus DE 698 33 579 T2 ist es bekannt, einen offenporigen Schaumstoffkörper, der auf eine Wunde gedrückt und sich in festem Kontakt mit einem Saugkopf befindet, mittels chirurgischer Abdecktücher an der Haut der Patienten zu fixieren. Die Abdecktücher weisen eine flexible Folie auf, deren Unterseite mit einer Klebstoffschicht beschichtet ist und deren Oberseite eine Schutzfolie trägt. Die Klebschicht ist mittels einer ablösbaren Schicht geschützt. Die ablösbare Schicht wird entfernt, wenn das Abdecktuch zum Einsatz kommt. Das Abdecktuch kann durch Zuschneiden an einen bestimmten Anwendungsfall angepasst werden.

Nach dem Stand der Technik ist es erforderlich, den Schaumstoffkörper und die Befestigungsmittel zum Fixieren des Schaumstoffkörpers an der Wunde getrennt zuzuschneiden. Das ist unbefriedigend, weil das getrennte Zuschneiden von Schaumstoffkörper und Befestigungsmittel mit einem höheren Zeitaufwand verbunden ist. Überdies steigt die Fehleranfälligkeit. Schließlich ist es schwierig, die Wundabdeckung an der Austrittstelle des Schlauches wirksam und dauerhaft abzudichten, wenn Schaumstoffkörper und Luftdichtung erst bei ihrer Anwendung zugeschnitten und aneinander befestigt werden. Andererseits ist es jedoch auch nicht möglich, auf eine Oberfläche des Schaumstoffkörpers eine selbstklebende Folie aufzubringen, die über die Oberfläche hinausgeht und dort mit einer ablösbaren Trennschicht beschichtet ist. Würde man solch eine Wundabdeckung zuschneiden, so müsste man auch die Abschnitte, die mit der Trennschicht verbunden sind, abschneiden, so dass die selbstklebende Folie nicht mehr zur Fixierung der Wundabdeckung genutzt werden könnte.

Aufgabe der Erfindung ist es, die Nachteile nach dem Stand der Technik zu beseitigen. Es soll insbesondere eine Wundabdeckung mit einem Schaumstoffkörper und einem darin verlaufenden Schlauchabschnitt angegeben werden, die sich aufwandsarm zuschneiden und handhaben lässt.

Diese Aufgabe wird durch die Merkmale der Ansprüche 1, 9 und 10 gelöst. Zweckmäßige Ausgestaltungen der Erfindungen ergeben sich aus den Merkmalen der Unteransprüche.

Nach Maßgabe der Erfindung ist eine Wundabdeckung vorgesehen, die einen offenporigen Schaumstoffkörper und ein schlauch- und/oder rohrförmiges Verbindungselement mit einem offenen Abschnitt umfasst, der in dem Schaumstoffkörper befestigt ist und an einer Oberseite des Schaumstoffkörpers in diesen eintritt, wobei die Oberseite des Schaumstoffkörpers mit einer Kunststofffolie bedeckt ist, die eine Öffnung zur Durchführung des Verbindungselementes aufweist und wobei die Kunststofffolie
- einen ersten Bereich, der an die Öffnung zur Durchführung des Verbindungselementes angrenzt und an der Oberseite des Schaumstoffkörpers befestigt ist,
- einen zweiten Bereich, der an den ersten Bereich angrenzt und gemeinsam mit dem ersten Bereich die Oberseite des Schaumstoffkörpers bedeckt, und
- einen dritten Bereich, der an den zweiten Bereich angrenzt und sich über die Oberseite des Schaumstoffkörpers hinaus erstreckt,
umfasst, wobei die Flächenseite der Kunststofffolie, die der Oberseite des Schaumstoffkörpers zugewandt ist, eine Haftmittelschicht aufweist, die im zweiten und dritten Bereich mit einer ablösbaren Trennschicht bedeckt ist.

Zur Anwendung der erfindungsgemäßen Wundabdeckung wird die Unterseite des Schaumstoffkörpers auf die Wundoberfläche oder auf eine andere Oberfläche, beispielsweise auf die Oberseite eines anderen Schaumstoffkörpers oder eines Schwammes, aufgelegt. Mittels der Kunststofffolie, die an der Oberseite des Schaumstoffkörpers in ihrem ersten Bereich befestigt ist, kann der Schaumstoffkörper an der Haut des Patienten außerhalb der Peripherie des Schaumstoffkörpers mittels der dritten Bereiche befestigt werden. Dabei kann folgendermaßen vorgegangen werden:

Zunächst wird der Schaumstoffkörper auf die vorgegebene Größe zugeschnitten. Dazu wird die Kunststofffolie in ihrem zweiten und dritten Bereich von der Oberseite des Schaumstoffkörpers getrennt. Dies ist möglich, weil zwischen der Oberseite des Schaumstoffkörpers und der Kunststofffolie in diesen beiden Bereichen die Trennschicht vorgesehen ist. Eine vollständige Trennung der Kunststofffolie von dem Schaumstoffkörper ist nicht möglich, weil die Kunststofffolie im ersten Bereich auf der Oberseite des Schaumstoffkörpers befestigt ist.

Muss die Kunststofffolie selbst nicht zugeschnitten werden, so kann nun der zugeschnittene Schaumstoffkörper mit seiner Unterseite auf die Wundoberfläche oder auf eine andere Oberfläche, beispielsweise auf die Oberseite eines anderen Schaumstoffkörpers oder eines Schwammes, aufgelegt werden. Muss hingegen auch die Kunststofffolie zu geschnitten werden, so kann diese mit einem oder mehreren weiteren Schnitten erfolgen, die durch den dritten Bereich der Kunststofffolie geführt werden.

Nach dem Zuschneiden des Schaumstoffkörpers und, falls erwünscht, der Kunststofffolie, wird die Trennschicht entfernt. Wird nun der dritte Bereich der Kunststofffolie an die Haut des Patienten angedrückt, so wird eine Klebeverbindung zwischen Haut und Kunststofffolie hergestellt, mit der der erfindungsgemäße Wundverband fixiert wird. Gleichzeitig gelangt der zweite Bereich der Kunststofffolie in Kontakt mit der Oberseite des Schaumstoffkörpers, so dass eine Klebeverbindung zwischen der Kunststofffolie und dem Schaumstoffkörper in diesem Bereich erreicht wird, was die Stabilität des erfindungsgemäßen Wundverbandes erhöht.

Die Oberseite des Schaumstoffkörpers, auf dem die Kunststofffolie in ihrem ersten Bereich befestigt ist, ist zweckmäßigerweise eine Flächenseite des Schaumstoffkörpers. Auch die Unterseite des Schaumstoffkörpers, die auf die Wunde oder auf eine andere Oberfläche, beispielsweise auf die Oberseite eines anderen Schaumstoffkörpers oder eines Schwammes, aufgelegt wird, ist vorzugsweise eine Flächenseite. Zweckmäßigerweise weist der Schaumstoffkörper zwei parallel und einander möglichst gegenüberliegende Flächenseiten auf, von denen die erste die Oberseite ist, an der das schlauch- und/oder rohrförmige Verbindungselement in den Schaumstoffkörper eintritt, und die zweite die Unterseite, die im Kontakt mit der Wunde gebracht wird. Der Schaumstoffkörper ist vorzugsweise im Wesentlichen prismen-, quader- oder würfelförmig. Er hat bevorzugt einen im Wesentlichen rechteckigen oder trapezförmigen Querschnitt. Besonders bevorzugt hat der Schaumstoffkörper eine Länge von 2 bis 30 cm, eine Breite von 2 bis 30 cm und eine Dicke von 1 bis 10 cm.

Der Schaumstoffkörper kann ein- oder mehrlagig sein. Im Falle eines mehrlagigen Schaumstoffkörpers verläuft der Abschnitt des schlauch- und/oder rohrförmigen Verbindungselementes, der in dem Schaumstoffkörper befestigt ist, zwischen zwei benachbarten Lagen.

Der Schaumstoffkörper besteht vorzugsweise aus einem Kunststoff. Der Kunststoff hat bevorzugt eine Porengröße von 10 bis 75 ppi. Dies entspricht 4 bis 25 Poren pro Zentimeter Oberfläche des Schaumstoffkörpers. Bevorzugt hat der Schaumstoffkörper 5 bis 20 Poren pro Zentimeter Oberfläche des Schaumstoffkörpers. Der offenporige Schaumstoffkörper hat vorzugsweise eine Rohdichte von 14 bis 75 kg/m³, bevorzugt eine Rohdichte von 20 bis 40 kg/m³. Unter Rohdichte wird hier die Dichte des Schaumstoffkörpers vor dem Eindringen von Wundsekret verstanden. Ein bevorzugter Kunststoff ist Polyurethan. Auch andere Kunststoffe sind geeignet, beispielsweise Polyvinylacetat.

Das schlauch- und/oder rohrförmige Verbindungselement dient zur Verbindung der erfindungsgemäßen Wundabdeckung mit einem Vakuummittel, beispielweise einer Saugpumpe. Das schlauch- und/oder rohrförmige Verbindungselement, das im Folgenden auch als Verbindungselement bezeichnet wird, ist vorzugsweise ein Schlauch, ein Rohr oder eine Kombination aus Schlauch- und Rohrsegmenten. Besonders bevorzugt ist das schlauch- und/oder rohrförmige Verbindungselement ein Schlauch. Unter einem Schlauch soll dabei eine flexible hohle Leitung verstanden werden. Der Schlauch besteht vorzugsweise aus Silikon oder einem anderen weichen, flexiblen und physiologisch akzeptablen Kunststoff. Unter einem Rohr wird eine starre hohle Leitung verstanden. Das Rohr ist vorzugsweise ein Kunststoffrohr, beispielweise ein Polycarbonatrohr, oder Metallrohr. Zweckmäßigerweise sind die beiden Enden des Verbindungselementes offene Enden.

Mittels des Verbindungselementes kann Unterdruck (Vakuum) in dem Schaumstoffkörper erzeugt werden. Auf diese Weise können mittels des Verbindungselementes Fluide, beispielsweise Wundsekrete aus einer Wunde, abgesaugt werden. Durch die Anwendung des Unterdrucks können derartige Fluide in den Schaumstoffkörper eintreten.

Nachfolgend werden Merkmale des Verbindungselementes am Beispiel eines Schlauches oder Rohres beschrieben. Ist das Verbindungselement eine Kombination aus Schlauch- und Rohrsegmenten, so kann diese Kombination ebenso eines oder mehrere dieser Merkmale aufweisen.

Zweckmäßigerweise weist der Schlauch- oder Rohrabschnitt, der in dem Schaumstoffkörper befestigt ist, eines der beiden offenen Enden des Schlauches oder Rohres auf. Er kann alternativ oder zusätzlich weitere Öffnungen aufweisen, die im Mantel des Schlauches oder Rohres ausgebildet sind. Aufgrund des offenen Endes und/oder der weiteren Öffnungen wird dieser Schlauch- oder Rohrabschnitt auch als "offener Abschnitt" bezeichnet.

Keine der Öffnungen sollte sich an einer Außenseite des Schaumstoffkörpers befinden. Zweckmäßigerweise sind die weiteren Öffnungen gleichmäßig über den Teil des Schlauch- oder Rohrabschnittes verteilt, der im Wesentlichen parallel zu der Oberseite verläuft, in der der Schlauch oder das Rohr in den Schaumstoffkörper eintritt. Die Öffnungen können in Abhängigkeit von dem Schlauch- oder Rohraußendurchmesser 1 bis 15 mm groß sein. Bevorzugt gleicht die Größe jeder Öffnung der Größe des offenen Endes des Schlauch- oder Rohrabschnittes. Alternativ kann vorgesehen sein, dass die Größe der weiteren Öffnungen mit zunehmender Entfernung von dem offenen Ende des Schlauch- oder Rohrabschnittes zunehmen kann. In diesem Fall sollte die größte weitere Öffnung so groß wie das offene Ende des Schlauches oder Rohres sein. Die weiteren Öffnungen sind vorzugsweise runde oder elliptische Öffnungen; sie können aber auch jede andere geeignete Form aufweisen. Der Abstand zwischen zwei benachbarten Öffnungen sowie der Öffnung, die dem offenen Ende des Teiles des Schlauches oder Rohres, der sich zwischen den beiden Lagen befindet, benachbart ist, sollte zwischen dem Zweifachen und dem Zehnfachen des Durchmessers der größten Öffnung liegen. In einer bevorzugten Ausführungsform sind die Öffnungen auf dem Schlauch- oder Rohrmantel versetzt zueinander angeordnet. Die Öffnungen sind vorzugsweise auf einer gedachten Spirale über den Schlauch- oder Rohrmantel verteilt.

Der Schlauch- oder Rohrabschnitt, der in dem Schaumstoffkörper befestigt ist, weist vorzugsweise Verzweigungen auf. Beispielsweise kann der Schlauch- oder Rohrabschnitt fächerförmig oder gabelförmig ausgebildet sein.

Der Schlauch- oder Rohrabschnitt weist vorzugsweise ein erstes Segment, dass sich von der Oberseite des Schaumstoffkörpers, in der der Schlauch oder das Rohr in den Schaumstoffkörper eintritt, bis etwa zu einem Punkt erstreckt, der gleichmäßig von dieser Oberseite und der Unterseite des Schaumstoffkörpers beabstandet ist. An diesem Punkt ist zweckmäßigerweise eine Krümmung des Schlauchabschnittes vorgesehen, an die sich ein zweites Segment anschließt, dessen Längsachse im Wesentlichen parallel zur Oberseite und/oder Unterseite des Schaumstoffkörpers verläuft. Sind Verzweigungen vorgesehen, so beginnen diese Verzweigungen vorzugsweise an dem zweiten Segment und erstrecken sich weiter bevorzugt in der Ebene der Längsachse des zweiten Segmentes, jedoch in Richtungen winklig, beispielsweise spitzwinkelig zur Längsachse des zweiten Segmentes. Auch die Verzweigungen selbst können sich verzweigen. Beispielsweise kann das zweite Segment einen oder mehrere, beispielsweise zwei Verzweigungspunkte aufweisen, von denen bevorzugt ein oder zwei Zweige abgehen.

Das zweite Segment des Schlauch- oder Rohrabschnittes ist vorzugsweise allseitig von dem Schaumstoffkörper umgeben, abgesehen von dem Übergang in das erste Segment.

Die Eintrittsstelle des Schlauches oder Rohres in den Schaumstoffkörper ist zweckmäßigerweise von allen Außenrändern der Oberseite des Schaumstoffkörpers beabstandet.

Der Schlauch- oder Rohrabschnitt, der in dem Schaumstoffkörper befestigt ist, ist zweckmäßigerweise luftdicht in den Schaumstoffkörper eingegossen oder eingeklebt.

Der Abschnitt des Verbindungselementes, der sich außerhalb des Schaumstoffkörpers befindet, sollte mit einer Einrichtung zur Erzeugung von Unterdruck, beispielsweise einer Saugpumpe, verbunden sein. Die Saugpumpe sollte einen Unterdruck zwischen 10 und 250 mm Hg erzeugen. Auf diese Weise kann ein Unterdruck erzeugt werden, so dass in der Wunde befindliches oder entstehendes Wundsekret in den Schaumstoffkörper eintritt, von dort über dessen Poren zu den Öffnungen des Abschnittes des Verbindungselementes, der in dem Schaumstoffkörper befestigt ist, gelangt und schließlich aus diesem Abschnitt des Verbindungselementes in den Abschnitt des Verbindungselementes gelangt, der sich außerhalb des Schaumstoffkörpers befindet. Die Saugpumpe kann jede Pumpe sein, die einen Unterdruck erzeugen kann, beispielsweise eine maschinelle Pumpe oder eine Handpumpe.

Die erfindungsgemäße Wundabdeckung kann mehr als ein Verbindungselement, beispielsweise bis zu fünf Verbindungselemente, umfassen. Die Verbindungselemente können unabhängig voneinander Schläuche, Rohre oder Kombinationen aus Schlauch- und Rohrsegmenten sein. Jedes dieser Verbindungselemente tritt durch eine separate Öffnung in den Schaumstoffkörper ein und weist jeweils einen Verbindungselement-Abschnitt auf, der in dem Schaumstoffkörper befestigt ist. Ebenso grenzen an jede dieser Öffnungen dritte Bereiche der Kunststofffolie an, die ineinander übergehen können.

Nachstehend wird die Kunststofffolie zur Vereinfachung am Beispiel einer erfindungsgemäßen Wundabdeckung mit einem Schlauch beschrieben. Anstelle des Schlauches kann auch ein anderes schlauch- und/oder rohrförmiges Verbindungselement vorgesehen sein.

Die Kunststofffolie ist vorzugsweise eine luftdichte Kunststofffolie. Auf diese Weise wird sichergestellt, dass bei Anwendung von Unterdruck keine Umgebungsluft angesaugt wird.

Der erste Bereich grenzt an die Öffnung zur Durchführung des Schlauches an und ist an der Oberseite des Schaumstoffkörpers befestigt, beispielsweise unter Verwendung eines Haftmittels. Zusätzlich kann der erste Bereich auch an dem Schlauch dichtend befestigt sein, um die Eintrittsstelle des Schlauches in den Schaumstoffkörper besser abzudichten. Wird bei der Herstellung der Wundabdeckung eine Kunststofffolie verwendet, die vollständig mit einem Haftmittel bedeckt ist und deren zweiter und dritter Bereich mit einer Trennschicht abgedeckt ist, so liegt das Haftmittel im ersten Bereich frei und kann so mit der Oberseite des Schaumstoffkörpers in einen Bereich, der an die Öffnung zur Durchführung des Schlauches angrenzt, verklebt werden.

An den ersten Bereich grenzt der zweite Bereich an. Dabei sollte sich der erste Bereich von der Öffnung zur Durchführung des Schlauches bis zu einer gedachten Umfangslinie erstrecken, die auf der Oberseite des Schaumstoffkörpers sowohl von der Öffnung zur Durchführung des Schlauches als auch von den Außenkanten der Oberseite beabstandet ist. Der zweite Bereich erstreckt sich vorzugsweise von dieser Umfangslinie bis zu den Außenrändern der Oberseite des Schaumstoffkörpers. Mit anderen Worten ist die Oberseite des Schaumstoffkörpers, wenn die Kunststofffolie auf der Oberseite aufliegt, außerhalb der Eintrittsöffnung des Schlauches vorzugsweise überall dort, wo sie nicht von dem ersten Bereich bedeckt ist, von dem zweiten Bereich bedeckt. Die Ausdehnung des ersten Bereiches ist so gewählt, dass eine feste und dauerhafte Fixierung der Kunststofffolie auf dem Schaumstoffkörper erreicht wird.

In einer Ausführungsform der Erfindung ist die Ausdehnung des ersten Bereiches so gewählt, dass er - abgesehen von der Eintrittsöffnung des Schlauches - den Schlauchabschnitt, der in dem Schaumstoffkörper verläuft, vollständig überdeckt. Projiziert man den Schlauchabschnitt mittels orthogonaler Parallelprojektion auf die Kunststofffolie, so liegt das Abbild des Schlauchabschnittes vollständig in der ersten Ebene der Kunststofffolie. Die Ausdehnung des ersten Bereiches kann jedoch auch geringer sein, solange eine dauerhafte Fixierung der Kunststofffolie auf dem Schaumstoffkörper sichergestellt ist.

Die Unterseite der Kunststofffolie, also die Flächenseite, die der Oberfläche des Schaumstoffkörpers zugewandt ist, ist im zweiten Bereich mit einem Haftmittel beschichtet, dass wiederum mit einer Trennschicht versehen ist, die das Anhaften des zweiten Bereiches der Kunststofffolie an dem Schaumstoffkörper vor Anwendung der erfindungsgemäßen Wundabdeckung verhindert.

An den zweiten Bereich grenzt der dritte Bereich an, der sich über die Oberseite des Schaumstoffkörpers hinaus erstreckt. Erstreckt sich der zweite Bereich bis zu den Außenrändern der Oberseite des Schaumstoffkörpers, so schließt sich dort der dritte Bereich an. Der dritte Bereich liegt bei Anwendung der erfindungsgemäßen Wundabdeckung nicht auf der Oberseite des Schaumstoffkörpers auf, sondern dient zur Befestigung der Wundabdeckung an der Haut außerhalb der Peripherie des Schaumstoffkörpers und vorzugsweise außerhalb der Wunde. Der dritte Bereich steht damit über die Außenränder der Oberseite über. Vorzugsweise überragt der dritte Bereich an zumindest einer, besonders bevorzugt an allen Außenrändern der Oberseite des Schaumstoffkörpers um 1 bis 20 cm, vorzugsweise um 1 bis 15 cm.

Aufgrund der Beschichtung der Unterseite der Kunststofffolie auch im dritten Bereich mit einem Haftmittel, kann die Kunststofffolie in ihrem dritten Bereich mit der Hautoberfläche verklebt werden. Vor der Anwendung der erfindungsgemäßen Wundabdeckung ist auch der dritte Abschnitt mit einer Trennschicht versehen, die das Anhaften des dritten Bereiches der Kunststofffolie vor Anwendung der erfindungsgemäßen Wundabdeckung verhindert. Der zweite und dritte Bereich haben vorzugsweise eine gemeinsame Trennschicht, so dass das Entfernen der Trennschicht gleich beide Bereiche freilegt, was die Anwendung der erfindungsgemäßen Wundabdeckung vereinfacht.

Bei der Kunststofffolie handelt es sich vorzugsweise um eine Polyvinylchlorid-Folie (PVC-Folie).

Bei dem Haftmittel kann es sich um bekannte Haftmittel handeln. Bevorzugt sind druckempfindliche Haftkleber. Der Haftkleber sollte in der Lage sein, eine Klebeverbindung zwischen der Kunststofffolie und dem Schaumstoffkörper einerseits und zwischen der Kunststofffolie und der Haut des Patienten herzustellen. In einer Ausführungsform der Erfindung enthält oder ist das Haftmittel ein oder mehrere Hydrokollode. In diesem Fall kann das Haftmittel auch ein Bindemittel enthalten.

Die Trennschicht verhindert das Ankleben des zweiten Bereiches an der Oberseite des Schaumstoffkörpers sowie des dritten Bereiches an beliebigen Oberflächen vor der Anwendung der erfindungsgemäßen Wundabdeckung. Bei der Trennschicht kann es sich um einen bekannten Release Liner handeln. Beispielsweise ist die Trennschicht aus einem Blattmaterial gebildet. Vorzugsweise ist sie eine Lage eines silikonierten Papiers.

Zum Abtrennen der Trennschicht kann vorgesehen sein, dass die Trennschicht an den Außenkanten der Kunststofffolie übersteht, um einen Griffbereich zu bilden. Die Trennschicht kann dann abgezogen werden. Dabei kann sie zerrissen werden. Alternativ kann die Trennschicht an einer Trennlinie in zwei Abschnitte trennbar sein, wobei sich die Trennlinie auf einer Linie befinden sollte, die den Schlauch an seiner Eintrittsstelle in den Schaumstoffkörper schneidet oder tangiert. Die Trennlinie kann durch einkerben, perforieren oder alternative Maßnahmen vorbestimmt sein.

Die erfindungsgemäße Wundabdeckung wird vorzugsweise steril verpackt. Die erfindungsgemäße Wundabdeckung ist insbesondere zur Ableitung von Wundsekret von großflächigen Wunden und aus Körperhöhlen geeignet. Sie ermöglicht eine Flächendrainage von Wundsekret.

Nach Maßgabe der Erfindung ist ferner eine Vorrichtung zum Absaugen von Sekreten aus Wunden vorgesehen, die die erfindungsgemäße Wundabdeckung sowie eine Saugpumpe umfasst, die mit dem Schlauch der Wundabdeckung in Wirkverbindung steht. An der Saugpumpe ist ein Ende des Schlauches angeschlossen. Von der Saugpumpe erstreckt sich ein Schlauchabschnitt, d. h. der Schlauchabschnitt der außerhalb des Schaumstoffkörpers verläuft bis zu der Eintrittsöffnung in den Schaumstoffkörper und geht dort in den Schlauchabschnitt über, der in dem Schaumstoffkörper befestigt ist. Die Eigenschaften der Saugpumpe und die Wirkungsweise der Kombination aus der erfindungsgemäßen Wundabdeckung und Saugpumpe sind oben beschrieben worden.

Schließlich ist nach Maßgabe der Erfindung ein Kit vorgesehen, das eine erfindungsgemäße Wundabdeckung und eine Saugpumpe, die mit dem Schlauch der Wundabdeckung in Wirkverbindung steht, umfasst.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine schematische Schnittdarstellung einer Ausführungsform der erfindungsgemäßen Wundabdeckung;
- Fig. 2: eine schematische Draufsicht der in Fig. 1 dargestellten Ausführungsform der erfindungsgemäßen Wundabdeckung;
- Fig. 3: eine vergrößerte Schnittdarstellung der in Fig. 1 dargestellten Ausführungsform der erfindungsgemäßen Wundabdeckung;
- Fig. 4: eine vergrößerte Schnittdarstellung einer anderen Ausführungsform der erfindungsgemäßen Wundabdeckung; und
- Fig. 5: eine schematische Darstellung einer erfindungsgemäßen Wundabdeckung, die auf eine Wunde aufgebracht ist.

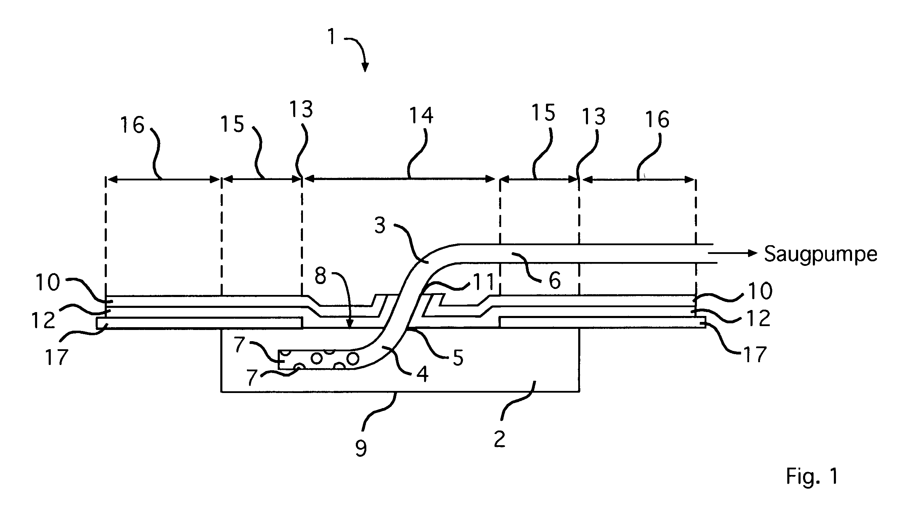

Die in den Figuren 1 bis 5 gezeigten Darstellungen sind nicht maßstabsgerecht. Insbesondere ist die Dicke der Folien im Vergleich zur Dicke des Schaumstoffkörpers und zum Außendurchmesser des Schlauches stark vergrößert.

Die in den Figuren 1 bis 3 gezeigte Ausführungsform der erfindungsgemäßen Wundabdeckung umfasst einen Schaumstoffkörper 2 und einen Schlauch 3 mit einem Schlauchabschnitt 4, der in dem Schaumstoffkörper 2 befestigt ist und an der Oberseite 8 des Schaumstoffkörpers 2 in diesen eintritt. Der Schaumstoffkörper 2 ist im Wesentlichen plattenförmig. Die Oberseite 8 des Schaumstoffkörpers ist mit einer luftdichten Kunststofffolie 10 bedeckt, die sich über die Außenränder der Oberseite 8 hinaus erstreckt. Die Unterseite 9 des Schaumstoffkörpers 2 ist zur Auflage auf eine Wunde oder auf eine andere Oberfläche, beispielsweise auf die Oberseite eines anderen Schaumstoffkörpers oder eine Schwammes, bestimmt.

Die Kunststofffolie 10 weist an der Eintrittsstelle 5 des Schlauches 3 in dem Schaumstoffkörper 2 eine Öffnung 11 zur Durchführung des Schlauches 4 auf. Die Kunststofffolie 10 weist drei Bereiche auf. Die Unterseite der Kunststofffolie 10, d.h. die Flächenseite, die der Oberseite des Schaumstoffkörpers zugewandt ist, ist mit einer Haftmittelschicht 12 beschichtet. Die Kunststofffolie 10 weist einen ersten Bereich 14 auf, der sich von der Öffnung 11 bis zu einer Umfangslinie 13 des ersten Bereiches 14 erstreckt, die sowohl von der Öffnung 11 als auch den Außenrändern des Schaumstoffkörpers 2 beabstandet ist. Dieser Bereich 14 ist unmittelbar mit der Oberfläche des Schaumstoffkörpers 2 verklebt und sorgt für eine dauerhafte Fixierung der Kunststofffolie 10 auf dem Schaumstoffkörper 2.

An den ersten Bereich 14 grenzt ein zweiter Bereich 15 an, der sich von der Umfangslinie 13 bis zum Außenrand des Schaumstoffkörpers 2 erstreckt. Dort beginnt der dritte Bereich 16, der über den Außenrand des Schaumstoffkörpers 2 hinausragt. Auf der Unterseite der Haftmittelschicht 12 ist im zweiten und dritten Bereich 15, 16 eine Trennschicht 17 aufgetragen. Die Trennschicht 17 kann über den dritten Bereich 16 hinausragen, wodurch die Ablösung der Trennschicht 17 erleichtert wird.

Es ist in den Figuren 1 bis 4 zu erkennen, dass der erste Bereich 14 und der zweite Bereich 14, abgesehen von der Öffnung 11, die Oberseite 8 des Schaumstoffkörpers 2 vollständig bedecken.

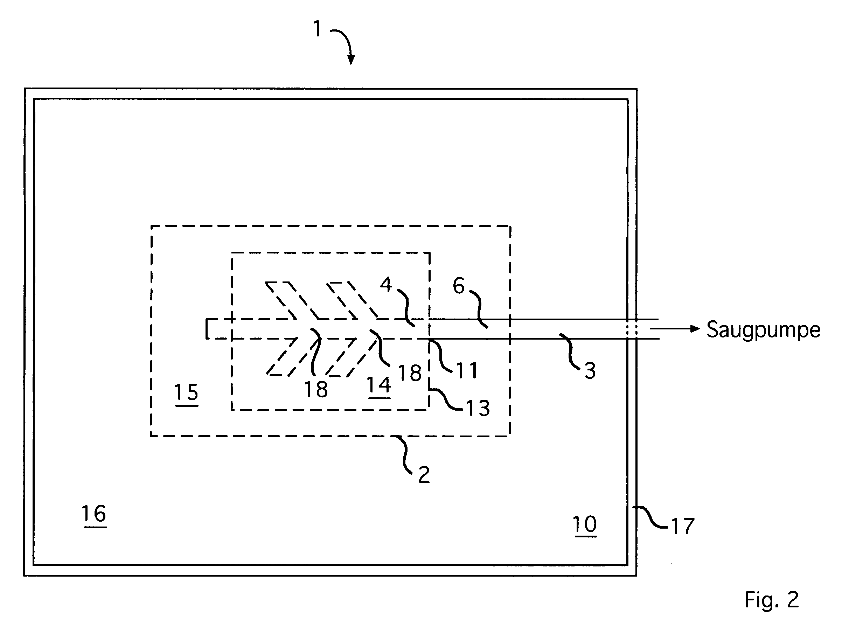

In Fig. 2 ist zu erkennen, dass der Schlauchabschnitt 4, der in dem Schaumstoffkörper 2 verläuft, Verzweigungen 18 aufweist.

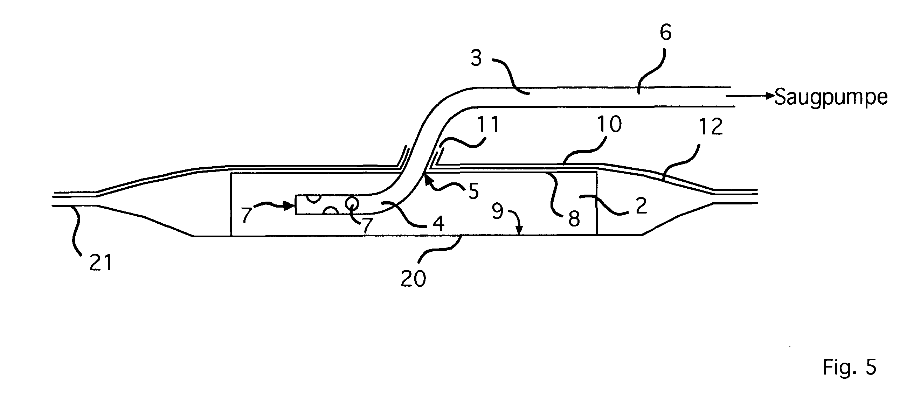

Zur Anwendung der erfindungsgemäßen Wundabdeckung 1 wird die Trennschicht 17 abgezogen. Dadurch wird die Haftmittelschicht 12 im zweiten und dritten Bereich 15, 16 freigelegt. Mittels der dritten Bereiche 16 wird die Kunststofffolie an der Haut 21 des Patienten unter Erhalt einer Klebverbindung befestigt, nachdem der Schaumstoffkörper 2 mit seiner Unterseite 9 auf eine Wunde 20 aufgelegt wurde (Fig. 5). Dabei wird der zweite Bereich mit der Oberseite 8 des Schaumstoffkörpers verklebt, wodurch eine sichere Fixierung der erfindungsgemäßen Wundabdeckung 1 auf der Wunde 20 erreicht wird. Wird nun über den Schlauch Vakuum mittels einer Saugpumpe auf die Wunde 20 angelegt, so werden Sekrete abgesaugt. Dabei sorgt die Kunststofffolie für einen luftdichten Verschluss.

Die Unterseite 9 kann jedoch auch auf einen anderen Schaumstoffkörper oder einen Schwamm aufgelegt werden.

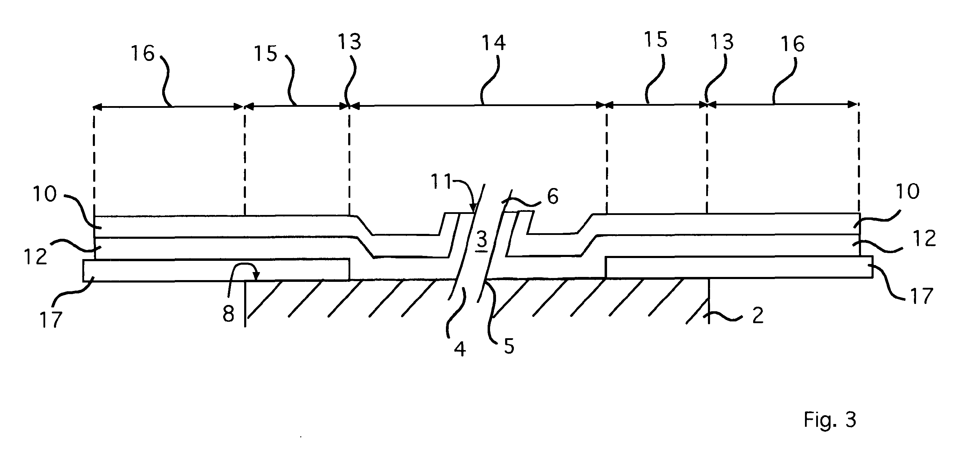

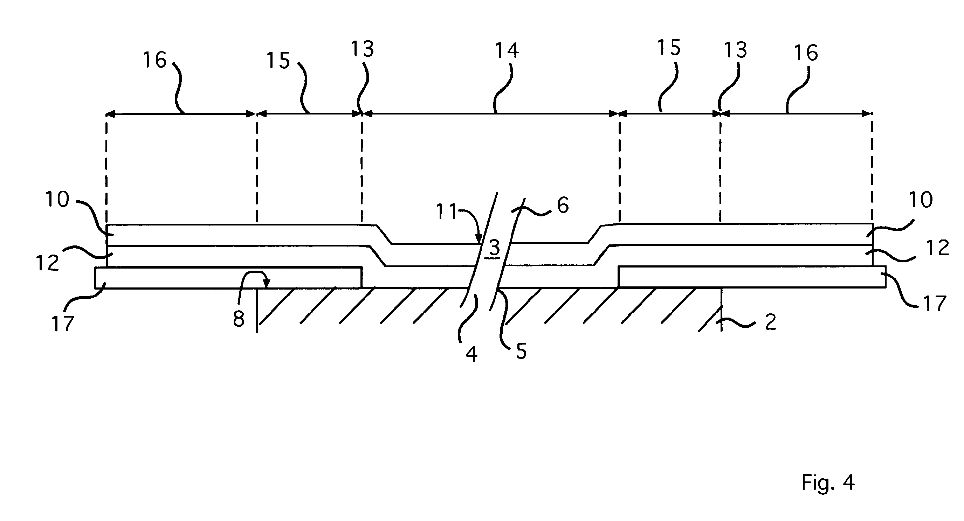

In Fig. 3 ist zu erkennen, dass die Kunststofffolie im ersten Bereich 14 auch mit dem äußeren Schlauchabschnitt 6 des Schlauches 3 verklebt sein kann, um eine sichere Abdichtung der Öffnung 11, durch die der Schlauch geführt ist, zu erreichen. In Fig. 4 ist hingegen eine Ausführungsform gezeigt, bei der der erste Bereich 14 zwar an den äußeren Schlauchabschnitt 6 an Öffnung 11 angrenzt, nicht aber mit diesem verklebt ist. Ansonsten entspricht diese Ausführungsform der in den Figuren 1 bis 3 gezeigten Ausführungsform.

### Bezugszeichenliste

- 1: Wundabdeckung
- 2: Schaumstoffkörper
- 3: Schlauch
- 4: innerer Schlauchabschnitt
- 5: Eintrittsstelle
- 6: äußere Schlauchabschnitt
- 7: Öffnungen im inneren Schlauchabschnitt
- 8: Oberseite des Schaumstoffkörpers
- 9: Unterseite des Schaumstoffkörpers
- 10: Kunststofffolie
- 11: Öffnung in der Kunststofffolie zur Durchführung des Schlauches
- 12: Haftmittelschicht
- 13: Umfangslinie
- 14: erster Bereich
- 15: zweiter Bereich
- 16: dritter Bereich
- 17: Trennschicht
- 18: Verzweigung

- 20: Wunde
- 21: Haut

## Patentansprüche

1. Wundabdeckung, umfassend einen offenporigen Schaumstoffkörper (2) und ein schlauch- und/oder rohrförmiges Verbindungselement (3) mit einem offenen Abschnitt (4), der in dem Schaumstoffkörper (2) befestigt ist und an einer Oberseite (8) des Schaumstoffkörpers (2) in diesen eintritt, **dadurch gekennzeichnet, dass** die Oberseite (8) des Schaumstoffkörpers (2) mit einer Kunststofffolie (10) bedeckt ist, die eine Öffnung (11) zur Durchführung des Verbindungselementes (3) aufweist, wobei die Kunststofffolie
- einen ersten Bereich (14), der an die Öffnung (11) zur Durchführung des Verbindungselementes (3) angrenzt und an der Oberseite (8) des Schaumstoffkörpers (2) befestigt ist,
- einen zweiten Bereich (15), der an den ersten Bereich (14) angrenzt und gemeinsam mit dem ersten Bereich (14) die Oberseite (8) des Schaumstoffkörpers (2) bedeckt, und
- einen dritten Bereich (16), der an den zweiten Bereich (15) angrenzt und sich über die Oberseite (8) des Schaumstoffkörpers (2) hinaus erstreckt,
umfasst, wobei die Flächenseite der Kunststofffolie (10), die der Oberseite (8) des Schaumstoffkörpers (2) zugewandt ist, eine Haftmittelschicht (12) aufweist, die im zweiten und dritten Bereich (15, 16) mit einer ablösbaren Trennschicht (17) bedeckt ist.

2. Wundabdeckung nach Anspruch 1, **dadurch gekennzeichnet, dass** die Kunststofffolie (10) luftdicht ist.

3. Wundabdeckung nach Anspruch 1 oder Anspruch 2, **dadurch gekennzeichnet, dass** der Schaumstoffkörper (2) zwei parallele Flächenseiten (8, 9) aufweist, von denen eine die Oberseite (8) ist, an der das Verbindungselement (3) in den Schaumstoffkörper (2) eintritt.

4. Wundabdeckung nach einem der vorstehenden Ansprüche, **dadurch gekennzeichnet, dass** die Haftmittelschicht (12) Hydrokolloide umfasst.

5. Wundabdeckung nach einem der vorstehenden Ansprüche, **dadurch gekennzeichnet, dass** der Abschnitt (4) des Verbindungselementes (3), der in dem Schaumstoffkörper (2) befestigt ist, Verzweigungen (18) aufweist, die jeweils mit zumindest einer Öffnung (7) versehen sind.

6. Wundabdeckung nach einem der vorstehenden Ansprüche, **dadurch gekennzeichnet, dass** die Kunststofffolie (10) zusätzlich an dem Verbindungselement (3) befestigt ist.

7. Wundabdeckung nach einem der vorstehenden Ansprüche, **dadurch gekennzeichnet, dass** die Trennschicht (17) aus einem Blattmaterial wie Papier gebildet ist.

8. Wundabdeckung nach einem der vorstehenden Ansprüche, **dadurch gekennzeichnet, dass** die Trennschicht (17) an einer Trennlinie in zwei Abschnitte trennbar ist, wobei sich die Trennlinie auf einer Linie befindet, die das Verbindungselement (3) an seiner Eintrittsstelle (5) in den Schaumstoffkörper (2) schneidet oder tangiert.

9. Vorrichtung zum Absaugen von Sekreten aus Wunden, umfassend eine Wundabdeckung (1) nach einem der Ansprüche 1 bis 7 und eine Saugpumpe, die mit dem Verbindungselement (3) der Wundabdeckung (1) in Wirkverbindung steht.

10. Kit, umfassend eine Wundabdeckung (1) nach einem der Ansprüche 1 bis 8 und eine Saugpumpe, die mit dem Verbindungselement (3) der Wundabdeckung (1) in Wirkverbindung steht.

## Claims

1. A wound covering comprising an open porous foam body (2) and a hose and/or tube-like connector (3) having an open section (4) fixed in the foam body (2) and entering the same at an upper surface (8) of the foam body (2), **characterized in that** the upper surface (8) of the foam body (2) is covered with a plastic foil (10) having an opening (11) for passing through the connector (3), wherein the plastic foil comprises
- a first region (14) bordering on the opening (11) for passing through the connector (3) and being fixed to the upper surface (8) of the foam body (2);
- a second region (15) bordering on the first region (14) and together with the first region (14) covering the upper surface (8) of the foam body (2); and
- a third region (16) bordering on the second region (15) and extending beyond the upper surface (8) of the foam body (2),
wherein the side of the plastic foil (10) facing the upper surface (8) of the foam body (2) has an adhesive layer (12) which in the second and third region (15, 16) is covered by a detachable interlayer (17).

2. The wound covering according to claim 1, **characterized in that** the plastic foil (10) is airtight.

3. The wound covering according to claim 1 or claim 2, **characterized in that** the foam body (2) has two parallel sides (8, 9), one of which is the upper surface (8) at which the connector (3) enters the foam body (2).

4. The wound covering according to any one of the preceding claims, **characterized in that** the adhesive layer (12) comprises hydrocolloids.

5. The wound covering according to any one of the preceding claims, **characterized in that** the section (4) of the connector (3) fixed in the foam body (2) has branches (18) each of which is provided with at least one opening (7).

6. The wound covering according to any one of the preceding claims, **characterized in that** the plastic foil (10) additionally is fixed to the connector (3).

7. The wound covering according to any one of the preceding claims, **characterized in that** the interlayer (17) is formed of a sheet material such as paper.

8. The wound covering according to any one of the preceding claims, **characterized in that** the interlayer (17) can be separated into two sections at an interface line, wherein the interface line is on a line that the connector (3) intersects or touches at its point of entry (5) into the foam body (2).

9. A device for aspirating secretions from wounds comprising a wound covering (1) according to any one of claims 1 to 7 and a suction pump that is actively connected to the connector (3) of the wound covering (1).

10. A kit comprising a wound covering (1) according to any one of claims 1 to 8 and a suction pump that is actively connected to the connector (3) of the wound covering (1).

## Revendications

1. Pansement comprenant un corps en mousse (2) à pores ouverts et un élément d'assemblage (3) en forme de tuyau et/ou tube avec une section ouverte (4) qui est fixée dans le corps en mousse (2) et y pénètre à une partie supérieure (8) du corps en mousse (2) **caractérisé en ce que** la partie supérieure (8) du corps en mousse (2) est couverte d'un film plastique (10) présentant une ouverture (11) pour passage de l'élément d'assemblage (3), le film plastique comprenant
- une première partie (14) étant adjacente à l'ouverture (11) pour le passage de l'élément d'assemblage (3) et étant fixée à la partie supérieure (8) du corps en mousse (2),
- une deuxième partie (15) étant adjacente à la première partie (14) et, avec la première partie (14), couvre la partie supérieure (8) du corps en mousse (2) et
- une troisième partie (16) étant adjacente la deuxième partie (15) et s'étendant au-delà de la partie supérieure (8) du corps en mousse (2),
la surface du film plastique (10) tournée vers la partie supérieure (8) du corps en mousse (2), présentant une couche adhésive (12) couverte dans la deuxième et la troisième parties (15, 16) couvertes d'une couche de séparation (17) dissoluble.

2. Pansement selon la revendication 1 **caractérisé en ce que** le film plastique (10) est étanche à l'air.

3. Pansement selon la revendication 1 ou 2 **caractérisé en ce que** le corps en mousse (2) présente deux surfaces parallèles (8, 9) dont l'une est la partie supérieure (8) à laquelle l'élément d'assemblage (3) pénètre dans le corps en mousse (2).

4. Pansement selon l'une des revendications précédentes **caractérisé en ce que** la couche adhésive (12) contient des hydrocolloïdes.

5. Pansement selon l'une des revendications précédentes **caractérisé en ce que** la partie (4) de l'élément d'assemblage (3) fixé dans le corps en mousse (2) présente des ramifications (18) pourvues au moins d'une ouverture (7).

6. Pansement selon l'une des revendications précédentes **caractérisé en ce que** le film plastique (10) est fixé en plus à l'élément d'assemblage (3).

7. Pansement selon l'une des revendications précédentes **caractérisé en ce que** la couche de séparation (17) est formée à partir d'un matériau de feuille comme du papier.

8. Pansement selon l'une des revendications précédentes **caractérisé en ce que** la couche de séparation (17) est séparable en deux parties à une ligne de séparation, la ligne de séparation se trouvant sur une ligne qui coupe ou touche l'élément d'assemblage (3) à son point d'entrée (5) dans le corps en mousse (2).

9. Dispositif d'aspiration de sécrétions venant des blessures comprenant un pansement (1) selon l'une des revendication 1 à 7 et une pompe d'aspiration qui interagit avec l'élément d'assemblage (3) du pansement (1).

10. Kit comprenant un pansement (1) selon l'une des revendication 1 à 8 et une pompe d'aspiration qui interagit avec l'élément d'assemblage (3) du pansement (1).
